(19) Europäisches Patentamt
European Patent Office
Office européen des brevets

(11) EP 0 896 534 B1

(12) **EUROPÄISCHE PATENTSCHRIFT**

(45) Veröffentlichungstag und Bekanntmachung des Hinweises auf die Patenterteilung:
**22.12.2004 Patentblatt 2004/52**

(21) Anmeldenummer: **97916406.8**

(22) Anmeldetag: **27.03.1997**

(51) Int Cl.$^7$: **A61K 31/365**, A61K 31/7048, A61K 47/14, A61K 47/44

(86) Internationale Anmeldenummer:
**PCT/EP1997/001569**

(87) Internationale Veröffentlichungsnummer:
**WO 1997/037653 (16.10.1997 Gazette 1997/44)**

(54) **INJEKTIONSFORMULIERUNGEN VON AVERMECTINEN UND MILBEMYCINEN AUF BASIS VON RIZINUSÖL**

INJECTION FORMULATIONS OF AVERMECTINS AND MILBEMYCINS BASED ON CASTOR OIL

FORMULATIONS A INJECTER D'AVERMECTINES ET DE MILBEMYCINES A BASE D'HUILE DE RICIN

(84) Benannte Vertragsstaaten:
**AT BE CH DE DK ES FR GB GR IE IT LI NL PT SE**

(30) Priorität: **09.04.1996 DE 19613972**

(43) Veröffentlichungstag der Anmeldung:
**17.02.1999 Patentblatt 1999/07**

(73) Patentinhaber: **Bayer HealthCare AG**
**51368 Leverkusen (DE)**

(72) Erfinder:
- **GROSSE-BLEY, Michael**
**D-51061 Köln (DE)**
- **KUJANEK, Richard**
**D-51061 Köln (DE)**

(74) Vertreter: **Petrovicki, Wolfgang, Dr.**
**Bayer AG**
**Konzernbereich RP**
**Patente und Lizenzen**
**51368 Leverkusen (DE)**

(56) Entgegenhaltungen:
**EP-A- 0 303 933** **WO-A-94/08566**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 0 896 534 B1

## Beschreibung

Die Erfindung betrifft neue Injektionsformulierungen von Avermectinen und Milbemycinen in Tiere auf Basis von Rizinusöl.

Injektionsformulierungen von Ivermectin sind bekannt aus EP-A 146 414. Die Formulierungen enthalten ein Lösemittelgemisch aus Propylenglykol und Glycerinformal im Verhältnis 60:40 V/V. Von Propylenglykol ist bekannt, daß es in bestimmten Konzentrationen lokale Unverträglichkeiten hervorrufen kann (siehe Review: B. Kruss, Acta Pharm. Technol. 35(4) (1989) 187-196). Auch kann es zur Ausfällung des wasserunlöslichen Wirkstoffs Ivermectin im Gewebe um die Applikationsstelle kommen. So wurden bei der Anwendung entsprechender Formulierungen deutliche Schwellungen und Gewebeunverträglichkeiten an den Injektionsstellen beobachtet, die sich zum Teil erst nach mehreren Wochen zurückbildeten.

Injektionsformulierungen bestimmter Avermectine sind bekannt aus EP-A 393 890. Es handelt sich um ölige Formulierungen auf Basis von Sesamöl und Ethyloleat im Verhältnis 90:10 V/V. Diese Formulierungen sind verträglich, haben aber den Nachteil, daß bei Lagerung im Kühlschrank bei 4°C bereits nach einigen Tagen ein wolkiger Niederschlag entsteht.

Weitere Injektionsformulierungen von Avermectinen sind bekannt aus EP-A 45 655. Die dort beschriebenen Formulierungen enthalten verhältnismäßig hohe Anteile an Emulgatoren und sind zum Teil wenig verträglich.

Injektionsformulierungen von Avermectinen, die Triacetin (Glycerintriacetat) enthalten, sind in EP-A 413 538 beschrieben. In EP-A 535 734 werden Injektionsformulierungen von Avermectinen auf Basis von Triacetin und hydriertem Rizinusöl beschrieben.

Weitere Formulierungen zur Injektion von Milbemycinen und Avermectinen sind in EP-A 525 307 beschrieben. Die Herstellung der Formulierungen erfolgt, indem Glycerintristearat mit dem Wirkstoff geschmolzen und mit einem öligen neutralen Triglycerid vermischt und unter Verwendung von z.B. Methylcellulose und Salzen emulgiert wird. Die durchschnittliche Partikelgröße in der so erhaltenen Mikroemulsion soll zwischen 25 und 300 µm liegen.

Die vorliegende Erfindung betrifft Injektionsformulierungen von Avermectinen und Milbemycinen auf Basis von Rizinusöl.

Die Formulierungen enthalten

1. Wirkstoff 0,1 bis 10 Gew.-%

2. Rizinusöl 15 bis 50 Gew.-%

3. Ein oder mehrere Co-Lösungsmittel aus der Reihe pflanzlicher oder synthetischer Fettsäureester ein- oder mehrwertiger Alkohole, aliphatischer oder aromatischer Alkohole, cyclischer Carbonate in Konzentrationen von mindestens 30 Gew.-%

4. gegebenenfalls weitere Hilfsstoffe.

Bevorzugt sind Formulierungen der folgenden Zusammensetzung:

0,1 bis 10 M/V eines Averrnectins oder Milbemycins in einem Lösungsmittel bestehend aus 15 bis 50 % V/V Rizinusöl, sowie mindestens 30 % V/V eines mittelkettigen Triglycerids und/oder Propylenglykol-octanoat/decanoat und/oder Ethyloleat und 0 bis 30 % V/V eines oder eines Gemisches aus den Lösungsmitteln Benzylalkohol, Propylenglykol oder Propylencarbonat, sowie gegebenenfalls bis zu 1000 ppm Stabilisatoren.

Besonders bevorzugt sind Formulierungen der folgenden Zusammensetzung:
0,1 bis 10 % Gew.% eines Avermectins oder Milbemycins, 20 bis 45 % V/V Rizinusöl, mindestens 45 % V/V mittelkettige Triglyceride oder Propylenglykoloctanoat/decanoat oder Ethyloleat und 0 bis 20 % V/V Benzylalkohol, 0 bis 10 % V/V Propylenglykol oder Propylencarbonat sowie gegebenenfalls bis zu 500 ppm Stabilisatoren.

Die erfindungsgemäßen Formulierungen weisen eine hervorragende Löslichkeit für die Wirkstoffe auf.

Die hohe Viskosität von Rizinusöl kann durch Zusatz von mittelkettigen Triglyceriden oder Propylenglykoloctanoat/decanoat oder Ethyloleat auf ein gewünschtes niedrigeres Maß eingestellt werden. Zusätzlich kann durch Addition von kleineren Volumina hydrophiler Lösemittel wie Benzylalkohol, Propylenglykol oder Propylencarbonat unter Beibehaltung eines einphasigen Systems die Löslichkeit des Wirkstoffs verbessert, die Viskosität weiter herabgesetzt und die Bioverfügbarkeit des Wirkstoffs verbessert werden. Die neuen Formulierungen sind außerordentlich gut verträglich und zeigen eine hohe Bioverfügbarkeit.

Die in den erfindungsgemäßen Formulierungen eingesetzten Wirkstoffe sind bekannt.

Avermectine wurden aus dem Mikroorganismus Streptomyces avermitilis als mikrobielle Metabolite isoliert (US-Pat. 4 310 519) und können im wesentlichen als Gemisch, bestehend aus den acht Komponenten $A_{1a}$, $A_{1b}$, $A_{2a}$, $A_{2b}$, $B_{1a}$, $B_{1b}$, $B_{2a}$ und $B_{2b}$, auftreten (I. Putter et al. Experentia 37 (1981) S. 963, Birkhäuser Verlag (Schweiz)). Daneben

besitzen auch die synthetischen Derivate, insbesondere das 22,23 Dihydroavermectin $B_1$ (Ivermectin), Interesse (US-Pat. 4 199 569). Milbemycin B-41 D wurde fermentativ aus Streptomyces hygroscopicus isoliert werden (vgl. "Milbemycin: Discovery and Development" I. Junya et al. Annu. Rep. Sankyo Res. Lab. 45 (1993), S. 1-98; JP-Pat. 8 378 549; GB 1 390 336).

Die Verwendung der Avermectine, z.B. 22,23 Dihydroavermectinen $B_1$ (Ivermectin) und Milbemycine als Endoparasitizide ist bekannt und Gegenstand zahlreicher Patentanmeldungen sowie Übersichtsartikel (z.B. Biologische Wirkungen in: "Ivermectin and Abamectin" W. C. Campbell, Ed., Springer Verlag, New York, N. Y., 1989; "Avermectins and Milbemycins Part II" H. G. Davies et al. Chem. Soc. Rev. 20 (1991) S. 271-339; Chemische Modifikationen in: G. Lukacs et al. (Eds.), Springer-Verlag, New York, (1990), Chapter 3; Cydectin ® [Moxidectin und Derivate]: G. T. Carter et al. J. Chem. Soc. Chem. Com-mun. (1987), S. 402-404); EP 423 445-A1) "Doramectin - a potent novel endectozide" A. C. Goudie et al. Vet. Parasitol. 49 (1993), S. 5-15).

Besonders hervorgehoben seien Avermectine und deren Derivate der allgemeinen Formel (I)

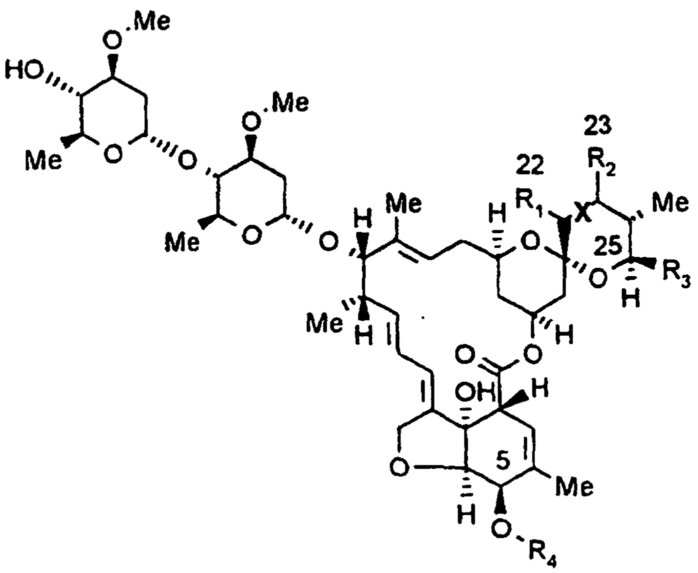

(I)

in welcher

die Reste $R^1$ bis $R^4$ die in der nachfolgenden Tabelle 1 angegebene Bedeutung haben und X für eine Einfach- oder Doppelbindung zwischen der $C_{22}$- und $C_{23}$-Position ($-C_{22}R^1-X-C_{23}R^2-$) stehen kann.

Im Falle einer Doppelbindung befinden sich keine Substituenten ($R^1$, $R^2$) an der $C_{22}$- und $C_{23}$-Position.

Tabelle 1

| **Makrocyclisches Lacton** | **$-C_{22}R^1-X-C_{23}R^2-$** | **$R^3$** | **$R^4$** |
|---|---|---|---|
| Avermectin $A_{1a}$ | -CH=CH- | -sec-Bu | -Me |
| Avermectin $A_{1b}$ | -CH=CH- | -iso-Pr | -Me |
| Avermectin $A_{2a}$ | $-CH_2-CHOH-$ | -sec-Bu | -Me |
| Avermectin $A_{2b}$ | $-CH_2-CHOH-$ | -iso-Pr | -Me |
| Avermectin $B_{1a}$ | -CH=CH- | -sec-Bu | -H |
| Avermectin $B_{1b}$ | -CH=CH- | -iso-Pr | -H |
| Avermectin $B_{2a}$ | $-CH_2-CHOH-$ | -sec-Bu | -H |
| Avermectin $B_{2b}$ | $-CH_2-CHOH-$ | -iso-Pr | -H |
| 22,23-Dihydroavermectin $B_{1a}$ | $-CH_2-CH_2-$ | -sec-Bu | -H |
| 22,23-Dihydroavermectin $B_{1b}$ | $-CH_2-CH_2-$ | -iso-Pr | -H |
| Doramectin | -CH=CH- | -Chx | -H |

Tabelle 1 (fortgesetzt)

| Makrocyclisches Lacton | $-C_{22}R^1-X-C_{23}R^2-$ | $R^3$ | $R^4$ |
|---|---|---|---|
| 22,23-Dihydroavermectin $B_1$ steht für Ivermectin; sec-Bu = sekundär Butyl; iso-Pr = Isopropyl; Chx = Cyclohexyl; -Me = Methyl | | | |

Die Avermectine und 22,23-Dihydroavermectine $B_1$ (Ivermectin) der allgemeinen Formel (I) werden in der Regel als Gemische eingesetzt. Von besonderem Interesse ist hierbei das Produkt Abamectin, das im wesentlichen die Avermectine $B_1$ enthält, und deren Hydrierungsprodukte, die 22,23-Dihydroavermectine $B_1$ (Ivermectin).

Die mit "b" bezeichneten Verbindungen der makrocyclischen Lactone, die in der $C_{25}$-Position einen iso-Propylrest besitzen, müssen nicht notwendigerweise von den "a" Verbindungen, welche eine sec-Butylgruppe in der $C_{25}$-Position haben, getrennt werden. Es wird generell das Gemisch beider Substanzen, bestehend aus > 80 % sec-Butylderivat ($B_{1a}$) und < 20 % iso-Propylderivat ($B_{1b}$) isoliert, und kann erfindungsgemäß verwendet werden. Zudem können bei den Stereoisomeren die Substituenten in der $C_{13}$- und $C_{23}$-Position sowohl α- als auch β-ständig am Ringsystem angeordnet sein, d. h. sich oberhalb oder unterhalb der Molekülebene befinden. In jedem Fall werden alle Stereoisomeren erfindungsgemäß berücksichtigt.

Besonders genannt seien die Milbemycine. Die Milbemycine haben die gleiche makrolide Ringstruktur wie die Avermectine oder 22,23-Dihydroavermectine $B_1$ (Ivermectin), tragen aber keinen Substituenten (d.h. fehlendes Oleandrose-Disaccharidfragment) in Position 13 ($R^5$ = Wasserstoff).

Beispielhaft seien als Milbemycine aus der Klasse der macrocyclischen Lactone die Verbindungen mit der allgemeinen Formel ( II ) genannt

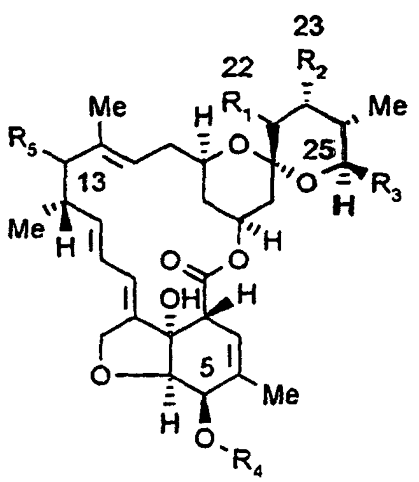

(II)

in welcher
die Reste $R^1$ bis $R^5$ die in der nachfolgenden Tabelle 2 angegebene Bedeutung haben:

Tabelle 2

| Makrocyclisches Lacton | $R^1$ | $R^2$ | $R^3$ | $R^4$ | $R^5$ |
|---|---|---|---|---|---|
| Milbemycin B41 D | -H | -H | -iso-Pr | -H | -H |
| Nemadectin | -H | -OH | 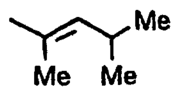 | -H | -H |
| Moxidectin | -H | =N-O-Me | 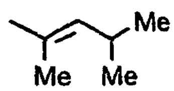 | -H | -H |

iso-Pr = Isopropyl

Ganz besonders hervorgehoben seien die Wirkstoffe

Avermectin $B_{1a}/B_{1b}$ (Ivermectin),

22,23-Dihydroavermectin $B_{1a}/B_{1b}$ (Ivermectin),

Doramectin,

Moxidectin.

Die Wirkstoffe liegen in den erfindungsgemäßen Formulierungen in Konzentrationen von 0,1 bis 10 Gew.-%, bevorzugt von 0,5-5 Gew.-%, besonders bevorzugt von 1-2 Gew.-% vor.

Das in den erfindungsgemäßen Formulierungen eingesetzte Rizinusöl ist bekannt. Es wird hier in Konzentrationen von 15 bis 50 Gew.-% verwendet.

Die in den erfindungsgemäßen Formulierungen eingesetzten Colösungsmittel sind bekannt.

Geeignete pflanzliche oder synthetische Fettsäureester mehrwertiger Alkohole (Öle) sind Fettsäuretriglyceride, vorzugsweise Fettsäuretriglyceride mit mittlerer Kettenlänge. Besonders eignen sich neutrale Öle, wie neutrale Pflanzenöle, und insbesondere fraktionierte Kokosnußöle, wie sie beispielsweise unter der Warenbezeichnung Miglyol bekannt und im Handel erhältlich sind, wozu erneut auf Lexikon der Hilfsstoffe, 3. Auflage, Seiten 808 bis 809, (1989) von Fiedler hingewiesen wird. Hierzu gehören beispielsweise: Miglyol 810: Hierbei handelt es sich um ein fraktioniertes Kokosnußöl, das Triglyceride von Caprylsäure und Caprinsäure enthält und ein Molekulargewicht von etwa 520 hat. Es weist eine Fettsäurezusammensetzung mit $C_6$ maximal 2 %, $C_8$ etwa 65 bis 75 %, $C_{10}$ etwa 25 bis 35 % und $C_{12}$ maximal 2 % auf, hat eine Säurezahl von etwa 0,1, verfügt über eine Verseifungszahl von etwa 340 bis 360 und verfügt über eine Iodzahl von maximal 1. Miglyol 812: Hierbei handelt es sich um ein fraktioniertes Kokosnußöl, das Triglyceride von Caprylsäure und Caprinsäure enthält und ein Molekulargewicht von etwa 520 hat. Es weist eine Fettsäurezusammensetzung mit $C_6$ maximal 3 %, $C_8$ etwa 50 bis 65 %, $C_{10}$ etwa 30 bis 45 % und $C_{12}$ maximal 5 % auf, hat eine Säurezahl von etwa 0,1, verfügt über eine Verseifungszahl von etwa 330 bis 345 und verfügt über eine Iodzahl von maximal 1. Miglyol 818: Triglyceride von Caprylsäure, Caprinsäure und Linolensäure mit einem Molekulargewicht von etwa 510. Es weist eine Fettsäurezusammensetzung mit $C_6$ maximal 3 %, $C_8$ etwa 45 bis 60 %, $C_{10}$ etwa 25 bis 40 %, $C_{12}$ etwa 2 bis 5 % und $C_{18:1}$ etwa 4 bis 6 auf, hat eine Säurezahl von etwa 0,2, verfügt über eine Verseifungszahl von etwa 315 bis 335 und verfügt über eine Iodzahl von maximal 10. Captex 355[(1)]: Triglycerid von Caprylsäure und Caprinsäure. Dieses Triglycerid weist einen Fettsäuregehalt an Capronsäure von etwa 2 %, an Caprylsäure von etwa 55 % und an Caprinsäure von etwa 42 % auf. Es hat eine Säurezahl von maximal 0,1, weist eine Verseifungszahl von maximal etwa 325 bis 340 auf und verfügt über eine Iodzahl von maximal 0,5. Ferner sind auch Triglyceride von Caprylsäure und Caprinsäure geeignet, wie die unter der Warenbezeichnung Myritol bekannten und im Handel erhältlichen Produkte, wozu beispielsweise auf Lexikon der Hilfsstoffe, 3. Auflage, Seite 834 (1989) von Fiedler hingewiesen wird. Das hierzu gehörende Produkt Myritol 813 hat eine Säurezahl von maximal 1, weist eine Verseifungszahl von etwa 340 bis 350 auf und verfügt über eine lodzahl von etwa 0,5.

Weiter geeignet sind: Monoglyceride, Diglyceride und Mono/Di-Glyceride, insbesondere Veresterungsprodukte von Caprylsäure oder Caprinsäure mit Glycerin. Bevorzugte Produkte dieser Klasse sind beispielsweise die Produkte, welche Monoglyceride und Diglyceride von Caprylsäure/Caprinsäure enthalten oder daraus im wesentlichen oder praktisch bestehen, und solche Produkte sind im Handel unter der Warenbezeichnung Imwitor erhältlich, wozu auf Lexikon der Hilfsstoffe, 3. Auflage, Seite 645 (1989) von Fiedler verwiesen wird. Ein besonders geeignetes Produkt aus dieser Klasse für die Anwendung in den erfindungsgemäßen Zusammensetzungen ist das Produkt Imwitor 742, bei dem es sich um ein Veiesterungsprodukt aus einem Gemisch von etwa 60 Gewichtsteilen (ppw) Caprylsäure und etwa 40 Gewichtsteilen (ppw) Caprinsäure mit Glycerin handelt. Imwitor 742 ist gewöhnlich eine gelbliche kristalline Masse, die bei etwa 26°C flüssig ist. Es weist eine Säurezahl von maximal 2 auf, hat eine Iodzahl von maximal 1, verfügt über eine Verseifungszahl von etwa 235 bis 275, enthält etwa 40 bis 50 % Monoglyceride, verfügt über einen Gehalt an freiem Glycerin von maximal 2 %, hat einen Schmelzpunkt von etwa 24 bis 26°C, enthält nichtverseifbare Bestandteile von maximal 0,3 % und verfügt über eine Peroxidzahl von maximal 1.

Sorbitanfettsäureester der verschiedensten bekannten Arten, wie sie beispielsweise unter der Warenbezeichnung Span im Handel erhältlich sind, und hierzu gehören beispielsweise Sorbitanmonolaurylester, Sorbitanmonopalmitylester, Sorbitanmonostearylester, Sorbitantristearylester, Sorbitanmonooleylester und Sorbitantrioleylester, und hierzu wird beispielsweise auf Lexikon der Hilfsstoffe, 3. Auflage, Seiten 1139 bis 1140 (1989) von Fiedler verwiesen.

Pentaerythritfettsäure und Polyalkylenglykolether, wie Pentaerythritdioleat, Pentaerythritdestearat, Pentaerythritmonolaurat, Pentaerythritpolyglykolether und Pentaerythritmonostearat und auch Pentaerythritfettsäureester, wozu auf Lexikon der Hilfsstoffe, 3. Auflage, Seiten 923 bis 924 (1989) von Fiedler verwiesen wird.

Monoglyceride, wie Glycerinmonooleat, Glycerinmonopalmitat und Glycerinmonostearat, wie sie beispielsweise unter den Warenbezeichnungen Myvatex, Myvaplex und Myverol bekannt und im Handel erhältlich sind, wozu auf Lexikon der Hilfsstoffe, 3. Auflage, Seite 836 (1989) von Fiedler verwiesen wird, und acetylierte, beispielsweise monoacetylierte und diacetylierte, Monoglyceride, wie sie beispielsweise unter der Warenbezeichnung Myvacet bekannt und im Handel erhältlich sind, wozu auf Lexikon der Hilfsstoffe, 3. Auflage, Seite 835 (1989) von Fiedler verwiesen wird.

Mono- und Difettsäureester von Propylenglykol, wie Propylenglykoldicaprylat, Propylenglykoldilaurat, Propylenglykolhydroxystearat, Propylenglykolisostearat, Propylenglykollaurat, Propylenglykolricinoleat, Propylenglykolstearat und dergleichen, wozu auf Lexikon der Hilfsstoffe, 3. Auflage, Seiten 1013 ff. (1989) von Fiedler hingewiesen wird. Besonders bevorzugt ist Propylenglykolcaprylsäurecaprinsäurediester, der unter der Warenbezeichnung Miglyol 840 bekannt und im Handel erhältlich ist, wozu auf Lexikon der Hilfsstoffe, 3. Auflage, Seite 809 (1989) von Fiedler verwiesen wird. Miglyol 840 hat einen Fettsäuregehalt von $C_6$ maximal etwa 3 Prozent, $C_8$ etwa 65 bis 80 Prozent, $C_{10}$ etwa 15 bis 30 Prozent und $C_{12}$ maximal 3 Prozent, und weist eine Säurezahl von maximal 0,1, eine Verseifungszahl von etwa 320 bis 340 und eine Iodzahl von maximal 1 auf.

Weitere geeignete Produkte dieser Klasse sind Capmul MCT(1), Captex 300(1), Captex 800(1), Neobee M5(2) und Mazol 1400(3) Imwitor(4)

Weitere Colösungsmittel sind Benzylalkohol, der gleichzeitig als Konservierungsmittel dienen kann, Alkohole wie Ethanol, Glykol, Glycerin, cyclische Carbonate wie Propylencarbonat. Die Colösungsmittel liegen in Konzentrationen von mindestens 30 Gew.-% vor.

Weitere Zusätze sind Stabilisatoren wie Butylhydroxyanisol (BHA), Butylhydroxytoluol (BHT) oder Propylgallat von insgesamt bis zu 1000 ppm. Besonders geeignete Stabilisatorkombinationen und -konzentrationen sind z.B. 100 ppm BHA oder 100 ppm BHA plus 150 ppm Propylgallat oder 200 ppm BHA plus 100 ppm Propylgallat.

Die Viskosität der erfindungsgemäßen Formulierungen liegt zwischen 25 bis 60 mPa.s (20°C), bevorzugt zwischen 30 bis 55 mPa.s (20°C), besonders bevorzugt zwischen 35 und 51 mPa.s (20°C).

Die Injektionsformulierungen von Avermectinen oder Milbemycinen auf Basis von Rizinusöl können hergestellt werden, indem man den Wirkstoff mit Rizinusöl mischt und die Colösungsmittel zufügt oder, indem man den Wirkstoff in einer Mischung aus Rizinusöl und den Colösungsmitteln auflöst.

Die folgenden Beispiele erläutern die Erfindung.

(1) = Capital City Products, P.O.Box 569, Columbus, OH, V.St.A.
(2) = Stepan, PVO Dept., 100 West Hunter Ave., Maywood, NJ 07607, V.St.A.
(3) = Mazer Chemicals, 3938 Porett Drive, Gurnee, IL, V.St.A,
(4) = Hüls AG, 14370 Marl, Deutschland

Anmerkung:

$$V/V = \frac{Volume}{Volume} \text{ entspricht Volumenprozent}$$

$$M/V = \frac{Masse}{Volume\ n}$$

1 % M/V heißt z.B. 10 mg Wirkstoff in 1 ml Lösung.

| **Beispiel 1** | | | | | |
|---|---|---|---|---|---|
| a) | Miglyol® 812<br>Rizinusöl<br>Benzylalkohol<br>Ivermectin<br><br>Dichte:<br><br>Viskosität: | q.s.100 % V/V<br>20 % V/V<br>2 % V/V<br>1 % M/V<br><br>0,954 g/ml<br><br>48 mPa.s bei 20°C<br>95 mPa.s bei 5°C | b) | Miglyol® 812<br>Rizinusöl<br>Benzylalkohol<br>Ivermectin<br><br>Dichte:<br><br>Viskosität: | q.s. 100 % V/V<br>20 % V/V<br>2 % V/V<br>2 % M/V<br><br>0,956 g/ml<br><br>48 mPa.s bei 20°C<br>105 mPa.s bei 5°C |

| **Beispiel 2** | | | | | |
|---|---|---|---|---|---|
| a) | Miglyol® 812<br>Rizinusöl<br>Propylencarbonat<br>Benzylalkohol<br>Ivermectin<br><br>Dichte:<br><br>Viskosität: | q.s.100 % V/V<br>20 % V/V<br>3 % V/V<br>2 % V/V<br>1 % M/V<br><br>0,962 g/ml<br><br>42 mPa.s bei 20°C<br>91 mPa.s bei 5°C | b) | Miglyol® 812<br>Rizinusöl<br>Propylencarbonat<br>Benzylalkohol<br>Ivermectin<br><br>Dichte:<br><br>Viskosität: | q.s.100 % V/V<br>20 % V/V<br>3 % V/V<br>2 % V/V<br>2 % M/V<br><br>0,964 g/ml<br><br>44 mPa.s bei 20°C<br>97 mPa.s bei 5°C |

| **Beispiel 3** | | | | | |
|---|---|---|---|---|---|
| a) | Miglyol® 812<br>Rizinusöl<br>Ivermectin<br><br>Dichte:<br><br>Viskosität: | q.s. 100% V/V<br>20 % V/V<br>1 % M/V<br><br>0,952 g/ml<br><br>51 mPa.s bei 20°C<br>105 mPa.s bei 5°C | b) | Miglyol® 812<br>Rizinusöl<br>Ivermectin<br><br>Dichte:<br><br>Viskosität: | q.s. 100 % V/V<br>20 % V/V<br>2 % M/V<br><br>0,954 g/ml<br><br>51 mPa.s bei 20°C<br>117 mPa.s bei 5°C |

| **Beispiel 4** | | | | | |
|---|---|---|---|---|---|
| a) | Miglyol® 812 | q.s. 100 % V/V | b) | Miglyol® 812 | q.s. 100 % V/V |
| | Rizinusöl | 35 % V/V | | Rizinusöl | 35 % V/V |
| | Ivermectin | 1 % M/V | | Ivermectin | 2 % M/V |
| | Dichte: | 0,939 g/ml | | Dichte: | 0,941 g/ml |
| | Viskosität: | 38 mPa.s bei 20°C<br>75 mPa.s bei 5°C | | Viskosität: | 42 mPa.s bei 20°C<br>76 mPa.s bei 5°C |

| **Beispiel 5** | | | | | |
|---|---|---|---|---|---|
| a) | Ethyloleat | q.s. 100 % V/V | b) | Ethyloleat | q.s. 100 % V/V |
| | Rizinusöl | 45 % V/V | | Rizinusöl | 45 % V/V |
| | Ivermectin | 1 % M/V | | Ivermectin | 2 % M/V |
| | Dichte: | 0,916 g/ml | | Dichte: | 0,918 g/ml |
| | Viskosität: | 40 mPa.s bei 20°C<br>91 mPa.s bei 5°C | | Viskosität: | 49 mPa.s bei 20°C<br>98 mPa.s bei 5°C |

| **Beispiel 6** | | | | | |
|---|---|---|---|---|---|
| a) | Miglyol® 840 | q.s.100 % V/V | b) | Miglyol® 840 | q.s 100 % V/V |
| | Rizinusöl | 35 % V/V | | Rizinusöl | 35 % V/V |
| | Propylenglykol | 5 % V/V | | Propylenglykol | 5 % V/V |
| | Benzylalkohol | 5 % V/V | | Benrylalkohol | 5 % V/V |
| | Ivermectin | 1 % M/V | | Ivermectin | 2 % M/V |
| | Dichte: | 0,952 g/ml | | Dichte: | 0,954 g/ml |
| | Viskosität: | 36 mPa.s bei 20°C<br>76 mPa.s bei 5°C | | Viskosität: | 38 mPa.s bei 20°C<br>81 mPa.s bei 5°C |

| **Beispiel 7** | | |
|---|---|---|
| a) | Ethyloleat | q.s. 100 % V/V |
| | Rizinusöl | 40 % V/V |
| | Propylenglykol | 5 % V/V |
| | Benzylalkohol | 5 % V/V |
| | Ivermectin | 1 % M/V |
| | Dichte: | 0,926 g/ml |

(fortgesetzt)

| Beispiel 7 | | |
|---|---|---|
| | Viskosität: | 34 mPa.s bei 20°C<br>70 mPa.s bei 5°C |

| Beispiel 8 | | |
|---|---|---|
| a) | Miglyol® 840<br>Rizinusöl<br>Benzylalkohol<br>Ivermectin<br><br>Dichte:<br><br>Viskosität: | q.s. 100 % V/V<br>35 % V/V<br>20 % VN<br>1 % M/V<br><br>0,965 g/ml<br><br>28 mPa.s bei 20°C<br>56 mPa.s bei 5°C |

| Beispiel 9 | | |
|---|---|---|
| a) | Miglyol® 840<br>Rizinusöl<br>Propylencarbonat<br>Benzylalkohol<br>Ivermectin<br>Dichte:<br><br>Viskosität | q.s. 100 % V/V<br>35 % V/V<br>10 % V/V<br>5 % V/V<br>1 % M/V<br>0,975 g/ml<br><br>27 mPa.s bei 20°C<br>53 mPa.s bei 5°C |

| Beispiel 10 | | |
|---|---|---|
| a) | Imwitor® 408<br>Rizinusöl<br>Ivermectin<br><br>Dichte<br><br>Viskosität | q.s. 100 % V/V<br>30 % V/V<br>1 % M/V<br><br>0,953 g/ml<br><br>30 mPa.s bei 20°C<br>66 mPa.s bei 5°C |

Imwitor® ist ein Markenname der Hüls AG. Bei Imwitor® 408 handelt es sich um 1,2-Propandiol-mono-dicaprylat (INCI (CTFA)-Bezeichnung). Laut vorläufiger Produktinformation enthält Imwitor® 408 ca. 10 % freies Propylenglykol und ca. 50 % Monoglyceride. Es zeigt ein hohes Lösungsvermögen für Ivermectin (>20 % M/V).

Allgemeine Herstellvorschrift für die Beispiele 1 bis 10 als sterile Lösungen zur Injektion:

Die Formulierhilfsstoffe werden in einen Edelstahlbehälter eingewogen und unter Rühren homogenisiert. Unter weiterem Rühren wird das Ivermectin eingebracht. Die Mischung wird auf 40 bis 50°C erwärmt, um die Auflösung

des Wirkstoffs zu beschleunigen (möglichst unter Stickstoffbegasung). Nach vollständiger Auflösung wird bei gleicher Temperatur über ein 0,22 µm Filter sterilfiltriert (in der Regel wird ein 0,45 µm oder 1 µm Filter vorgeschaltet). Es folgt aseptische Abfüllung in Braunglasflaschen.

Die so hergestellten Formulierungen sind bei der Anwendung am Rind hervorragend verträglich. Sie sind außerdem über mindestens 6 Wochen bei Temperaturen zwischen 4°C und 60°C lagerstabil.

## Patentansprüche

**1.** Formulierungen enthaltend:

1. Ein Avermectin oder Milbemycin als Wirkstoff 0,1 bis 10 Gew.-%,

2. Rizinusöl 15 bis 50 Gew.-%,

3. Co-Lösungsmittel aus der Reihe pflanzlicher oder synthetischer Fettsäureester ein- oder mehrwertiger Alkohole, aliphatischer oder aromatischer Alkohole, cyclischer Carbonate in Konzentrationen von mindestens 30 Gew.-%,

4. gegebenenfalls weitere Hilfsstoffe.

**2.** Injektionsformulierungen von Avermectinen oder Milbemycinen auf Basis von Rizinusöl der folgenden Zusammensetzung:

0,1 bis 10 % M/V eines Avermectins oder Milbemycins in einem Lösungsmittel bestehend aus 15 bis 50 % V/V Rizinusöl, sowie mindestens 30 % V/V eines mittelkettigen Triglycerids und/oder Propylenglykoloctanoat/decanoat und/oder Ethyloleat und 0 bis 30 % V/V eines oder eines Gemisches aus den Lösungsmitteln Benzylalkohol, Propylenglykol oder Propylencarbonat, sowie gegebenenfalls bis zu 1000 ppm Stabilisatoren.

**3.** Formulierungen von Avermectinen oder Milbemycinen auf Basis von Rizinusöl der folgenden Zusammensetzung:

0,1 bis 10 Gew.-% eines Avermectins oder Milbemycins, 20 bis 45 % V/V Rizinusöl, mindestens 45 % V/V mittelkettige Triglyceride oder Propylenglykol-octanoat/decanoat oder Ethyloleat und 0 bis 20 % V/V Benzylalkohol, 0 bis 10 % V/V Propylenglykol oder Propylencarbonat sowie gegebenenfalls bis zu 500 ppm Stabilisatoren.

## Claims

**1.** Formulations comprising:

1. an avermectin or milbemycin as active compound 0.1 to 10% by weight,

2. castor oil 15 to 50% by weight,

3. co-solvents from the series consisting of vegetable or synthetic fatty acid esters of mono- or polyhydric alcohols, aliphatic or aromatic alcohols, cyclic carbonates in concentrations of at least 30% by weight,

4. if appropriate, further auxiliaries.

**2.** Injection formulations of avermectins or milbemycins based on caster oil, of the following composition:

0.1 to 10% m/v of an avermectin or milbemycin in a solvent mixture consisting of 15 to 50% v/v of castor oil, and at least 30% v/v of medium-chain triglyceride and/or propylene glycol octanoate/decanoate and/or ethyl oleate and 0 to 30% v/v of one or of a mixture of the solvents benzyl alcohol, propylene glycol or propylene carbonate, and optionally up to 1000 ppm of stabilizers.

**3.** Formulations of avermectins or milbemycins based on caster oil, of the following composition:

0.1 to 10% by weight of an avermectin or milbemycin, 20 to 45% v/v of castor oil, at least 45% v/v of medium-chain triglycerides or propylene glycol octanoate/decanoate or ethyl oleate and 0 to 20% v/v of benzyl alcohol, 0 to 10% v/v of propylene glycol or propylene carbonate and optionally up to 500 ppm of stabilizers.

## Revendications

**1.** Formulations contenant :

1. une avermectine ou une milbémycine comme substance active en proportion de 0,1 à 10 % en poids,
2. de l'huile de ricin en proportion de 15 à 50 % en poids,
3. des cosolvants de la série d'esters d'acides gras végétaux ou synthétiques d'alcools monovalents ou polyvalents, d'alcools aliphatiques ou aromatiques, de carbonates cycliques à des concentrations d'au moins 30 % en poids,
4. éventuellement d'autres substances auxiliaires.

**2.** Formulations injectables d'avermectines ou de milbémycines à base d'huile de ricin, de composition suivante :

0,1 à 10 % en masse/volume d'une avermectine ou d'une milbémycine dans un solvant constitué de 15 à 50 % en volume/volume d'huile de ricin, ainsi qu'au moins 30 % en volume/volume d'un triglycéride à moyenne longueur de chaîne et/ou d'octanoate/décanoate de propylèneglycol et/ou d'oléate d'éthyle et 0 à 30 % en volume/volume d'un solvant ou d'un mélange de solvants choisis entre l'alcool benzylique, le propylèneglycol ou le carbonate de propylène, ainsi que, le cas échéant, jusqu'à 1000 ppm d'agents stabilisants.

**3.** Formulations d'avermectines ou de milbémycines à base d'huile de ricin, de composition suivante :

0,1 à 10 % en poids d'avermectine ou de milbémycine, 20 à 45 % en volume/volume d'huile de ricin, au moins 45 % en volume/volume de triglycérides de moyenne longueur de chaîne ou d'octanoate/décanoate de propylèneglycol ou d'oléate d'éthyle et 0 à 20 % en volume/volume d'alcool benzylique, 0 à 10 % en volume/volume de propylèneglycol ou de carbonate de propylène ainsi que, le cas échéant, jusqu'à 500 ppm d'agents stabilisants.